Europäisches Patentamt

European Patent Office

Office européen des brevets

(11) Numéro de publication: **0 061 228**
**A2**

(12) **DEMANDE DE BREVET EUROPEEN**

(21) Numéro de dépôt: **82200340.6**

(22) Date de dépôt: **18.03.82**

(51) Int. Cl.³: **C 12 M 1/00**

(30) Priorité: **19.03.81 BE 204178**

(43) Date de publication de la demande:
**29.09.82 Bulletin 82/39**

(84) Etats contractants désignés:
**AT BE CH DE FR GB IT LI LU NL SE**

(71) Demandeur: **ATELIERS DE CONSTRUCTIONS
ELECTRIQUES DE CHARLEROI (ACEC) Société Anonyme
54, Chaussée de Charleroi
B-1060 Bruxelles(BE)**

(72) Inventeur: **Mullier, Philippe
30, Chaussée de Wavre
B-5800 Gembloux(BE)**

(74) Mandataire: **Bossard, Franz et al,
ACEC - Service des Brevets Boîte Postale 4
B-6000 Charleroi(BE)**

(54) **Cuve de réaction pour réactif liquide.**

(57) Une cuve de réaction comprend une tuyauterie d'amenée d'un liquide réactif au bas d'une chambre primaire de décantation surmontant un lit d'un catalysateur physicochimique ou biologique, au moins un plan déflecteur incliné faisant partie d'une hotte de gaz et recouvrant partiellement la chambre primaire de décantation, une chambre secondaire de décantation adossée à au moins un des plans déflecteurs inclinés et une tuyauterie d'évacuation dans la partie supérieure de la chambre secondaire de décantation; cette chambre secondaire de décantation est subdivisée en un grand nombre d'espaces inclinés de faible hauteur obtenus au moyen d'au moins un faisceau de lames inclinées conduisant le liquide réactif vers le haut dans au moins un espace collecteur relié à la tuyauterie d'évacuation, au moins un déflecteur incliné est disposé en dessous des ouvertures d'entrée de dits espaces inclinés.

./...

Fig.2

## CUVE DE RÉACTION POUR RÉACTIF LIQUIDE.

Il existe déjà des cuves de réaction pour réactif liquide traversant un lit d'un catalyseur physico-chimique ou biologique de faible densité et de dimensions particuliaires au moins partiellement fluidisés par du gaz engendré par la réaction dans lequel le catalyseur en-entraîné par le liquide est récupéré.

L'invention a pour but un réacteur hétérogène multiphasique à récupération sélective de catalyseur, sans nécessiter des organes qui demandent un entretien systématique ou doivent être entraînés en permanence ou par intermittences par une source d'énergie extérieure et dans lequel une abrasion d'un catalyseur fixé sur un support est évité.

L'invention est applicable dans le cas de réactions biochimiques inhibées par le substrat et dans le cas où des vitesses de conversion élevées doivent être obtenues. Dans de tels cas, l'invention a pour but une cuve divisée en deux zones de régimes hydrodynamiques multiphasiques différents: une première zone à phase mélangée permettant d'éviter l'inhibition par le substrat et une deuxième zone dans laquelle la vitesse de réaction par unité de volume peut être optimisé.

La cuve de réaction suivant l'invention comprend une tuyauterie d'amenée d'un liquide réactif au bas d'une chambre primaire de décantation surmontant un lit d'un catalysateur physico-chimique ou biologique, au moins un plan déflecteur incliné faisant partie d'une hotte collectrice de gaz et recouvrant partiellement la chambre primaire de décantation, une chambre secondaire de décantation adossée à au moins un des plans déflecteurs inclinés et une tuyauterie d'évacuation dans la partie supérieure de la chambre secondaire de décantation; elle est caractérisée en ce que la chambre secondaire de décantation est subdivisée en un grand nombre d'espaces inclinées de faible hauteur obtenus au moyen d'au moins un faisceau de lames inclinées conduisant le liquide réactif vers le haut dans au moins un espace collecteur relié à la tuyauterie d'évacuation et en ce qu'au moins un déflecteur incliné est disposé en dessous des ouvertures d'entrée de dits espaces inclinées.

La cuve suivant l'invention possède l'avantage que le liquide réactif monte lentement dans un mouvement ascendant vers le dessus de la cuve, sans être obligé de jamais redescendre. Malgré cela, et ce grâce aux espaces inclinés de faible hauteur, une rapide et excellente séparation entre liquide et parties lourdes a lieu, et tandis que le liquide poursuit son mouvement ascendant, les parties lourdes descendent le long des lamelles inclinées en formant une boue lourde résistant à l'entraînement par le liquide ascendant.

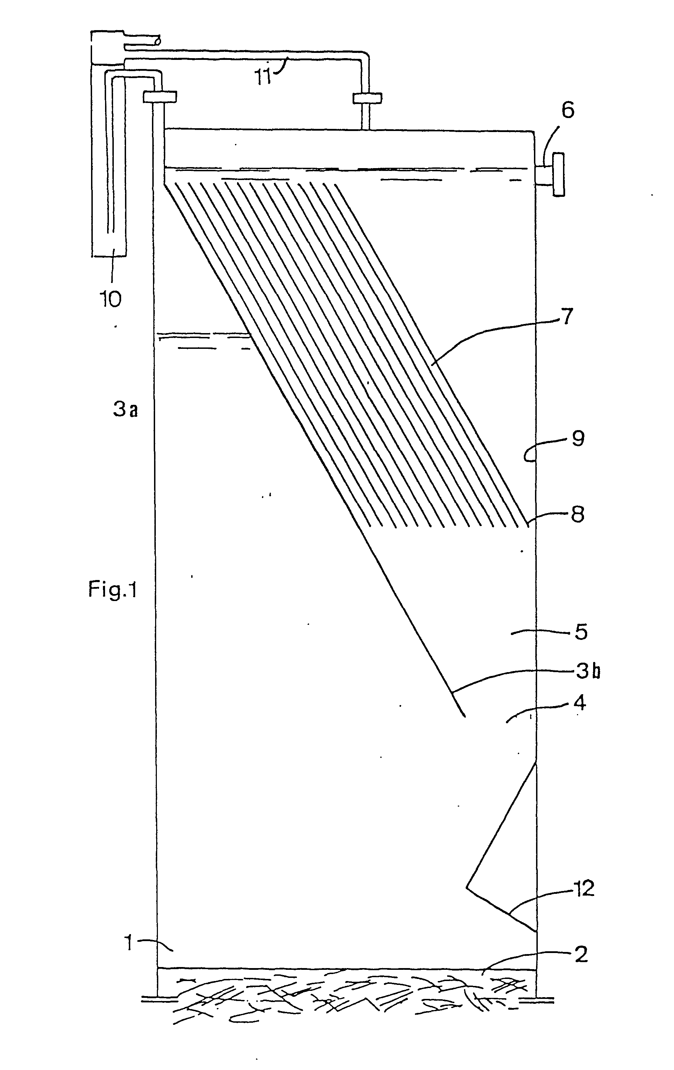

L'invention est décrite ci-dessous par rapport à quelques exemples de formes d'exécuiton représentés aux figures 1 à 3 du dessin annexé.

La figure 1 du dessin représente une coupe schématique d'une cuve deréaction suivant l'invention. Une tuyauterie d'amenée, non représentée, alimente en liquide réactif, par exemple en eau résiduaire une chambre primaire parallélépipédique de décantation 1 garnie d'un lit d'un catalyseur par exemple un catalyseur biologique 2. Un plan déflecteur incliné 3a, 3bfaisant partie d'une hotte collectrice de gaz recouvre la chambre primaire de décantation 1 sauf à l'endroit d'une ouverture 4 le long du bord horizontal inférieur du plan incliné 3a, 3b. Une chambre de décantation secondaire 5 est adossée à la partie supérieure 3a du dit plan déflecteur incliné et est disposée au dessus de la dite ouverture 4. Une tuyauterie d'évacuation 6 est prévue à la partie supérieure de cette chambre secondaire de décantation 5.

Le plan incliné 3a, 3b inclut avec l'horizontale par exemple un angle de 50° à 65° et un faisceau de lamelles 7 parallèle à ce plan incliné 3a, 3b est adossé à ce dernier. Le bord inférieur horizontal 8 de la dernière lamelle du faisceau, celle voisine de la paroi verticale 9 de la cuve est de préférence distante de cette dernière d'une valeur inférieure ou approximativement égale à la distance entre deux lamelles voisines du faisceau 7. La longueur des lamelles du faisceau et l'écartement entre lamelles voisines sont choisies en fonction du pouvoir de séparation désiré. Dans l'exemple représenté, la distance qui sépare le bord horizontal supérieur du plan incliné 3a, 3b du bord horizontal supérieur de la dernière lamelle du faisceau est approximativement égale à la moitié de la distance entre le bord horizontal supérieur du plan incliné et la paroi verticale 9 du bac.

La largeur de l'ouverture 4 au bas de la chambre secondaire de décantation secondaire 5 est choisie relativement grande puisque la quantité totale du liquide réactif doit la traverser. Dans l'exemple choisi, cette largeur vaut environ la moitié de l'épaisseur du faisceau des lamelles mesurée perpendiculairement aux lamelles.

Le gaz collecté sous la hotte constituée par le plan incliné 3a, 3b est amené par exemple au bas d'une colonne d'eau 10, tandis qu'un conduit 11 amène le gaz dégagé dans la chambre secondaire de décantation 5 au dessus de cette colonne 10. Ainsi une pression de gaz réglable par le choix de la hauteur d'eau dans la colonne 10 est maintenue sous la hotte formée par le plan déflecteur incliné 3a, 3b et la paroi 9.

En dessous de l'ouverture 4, un déflecteur 12 inférieur, coudé dévie vers la hotte sous le plan incliné 3a, 3b les bulles de gaz montantes et permet l'écoulement par gravité des boues de décantation qui descendent de la chambre secondaire de décantation 5.

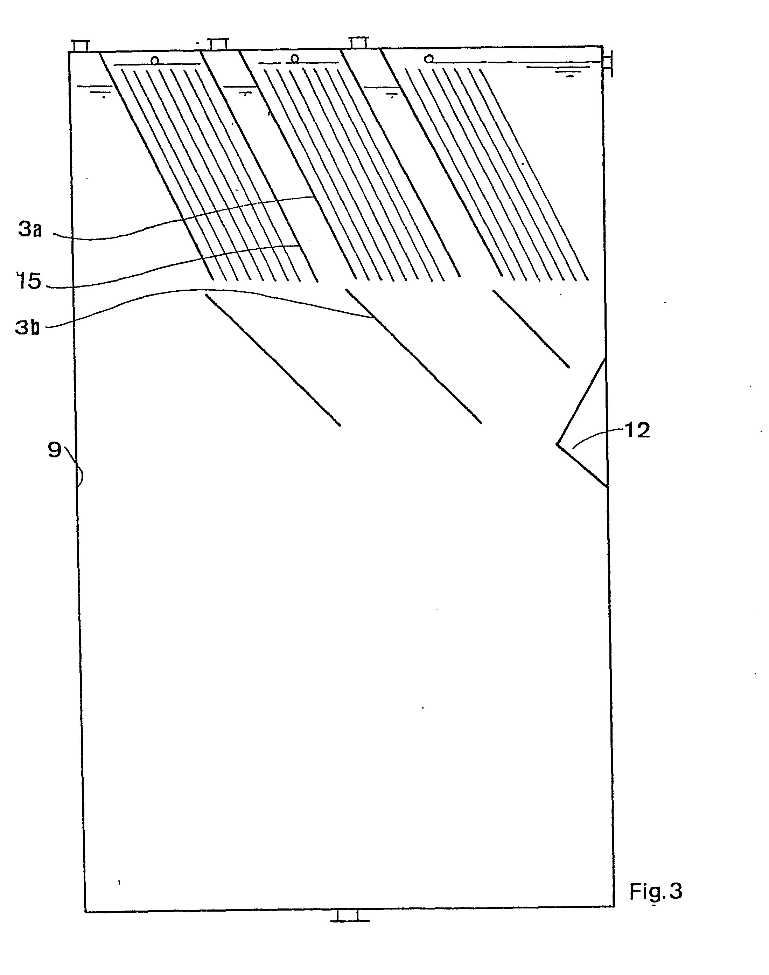

Le liquide qui monte dans les espaces inclinés entre les lamelles du faisceau 7 conserve une vitesse constante, tandis que le liquide qui monte dans l'espace délimité par la paroi 9 du bac et la dernière lamelle du faisceau 7 possède une vitesse environ égale à celle du liquide entre les lamelles au niveau du bord inférieur horizontal 8 de la dernière lamelle, mais au fur et à mesure qu'il monte dans la chambre 5, sa vitesse diminue. La dimension de l'ouverture entre le bord 8 et la paroi 9 est choisie de telle manière que l'efficacité de décantation et donc de récupération du catalyseur biologique ou physicochimique obtenu dans cet espace est sensiblement du même ordre de grandeur que celui obtenu dans l'espace entre les lamelles. Il va de soi que dans ces conditions l'espace entre la paroi 9 et la dernière lamelle du faisceau est mal utilisé et on donnera dans un exemple ci-dessous, montré à la figure 3, un moyen de maintenir cet espace relativement petit.

Les lamelles du faisceau 7 peuvent être constituées de plans ou d'ondulés par exemple en verre, polypropylène, polyéthylène, chlorure de polyvinyle ou stratifiés renforcés de fibres de verre recouvertes de couches superficielles en ces matériaux ou d'un agent hydrofuge tel qu'un vernis à base de méthyl-silane.

En cas d'utilisation d'ondulés ou de profils non plans pour les lamelles, les plans mentionnés dans la description ci-dessus concernent les plans moyens des dites lamelles.

Dans la chambre primaire de décantation, un régime de mélange parfait peut être obtenu pour la phase liquide par l'optimisation des débits relatifs entre phases liquide et gazeuse. Ces débits peuvent être ajustés l'un par rapport à l'autre au moyen d'un dosage par l'intermédiaire de conduits et d'une pompe de recyclage, non représentés, disposés entre la tuyauterie d'évacuation 6 et la tuyauterie d'amenée.

Dans la chambre secondaire de décantation, un régime hydrodynamique "piston" est assuré par la subdivision de l'espace au moyen des faisceaux de lamelles. Cette subdivision a pour effet d'interdire des mouvement turbulants du liquide et d'assurer son écoulement avec une régularité similaire à celle qui pourrait être obtenue si des pistons le forçaent à travers les espaces entre les lamelles.

La longueur des canaux entre les lamelles et la section de chaque canal sont déterminées en fonction de la vitesse du liquide réactif et du diamètre de coupure des particules, c'est-à-dire le diamètre limite des particules en dessous duquel une particule est entraînée vers le haut et au dessus duquel une particule amorce un mouvement de sédimentation vers le bas.

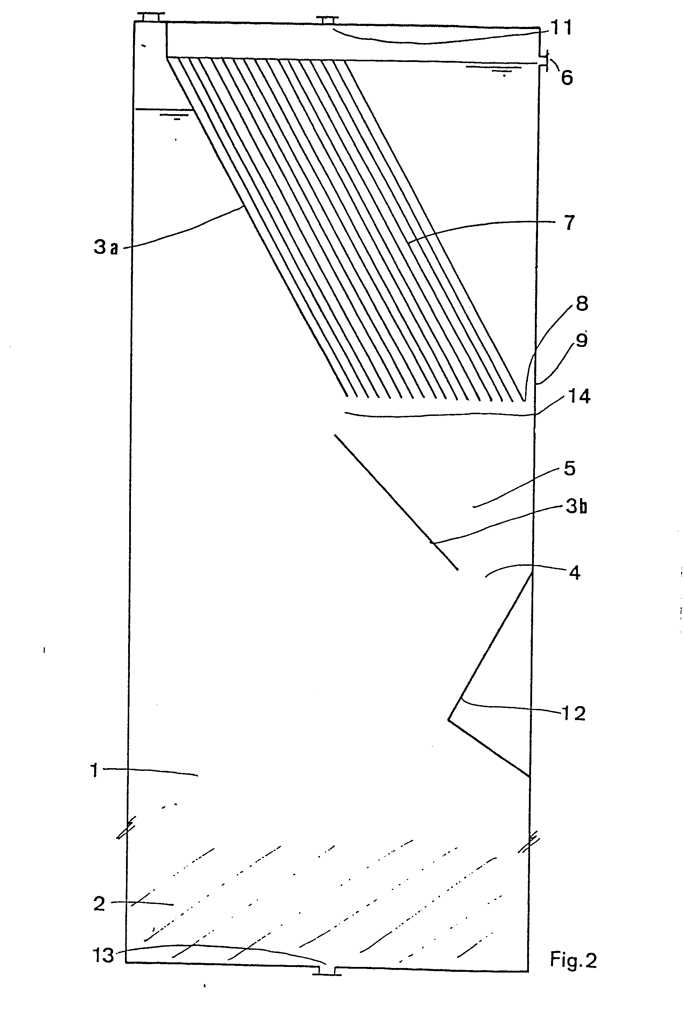

A la figure 2 une variante de la cuve selon la figure 1 est représentée. Ici, la tuyauterie d'amenée 13 est montrée au fond de la cuve 1. La seule différence par rapport à la fig. 1 est le fait que le plan déflecteur incliné est subdivisé en deux plans 3a et 3b ménageant non pas une ouverture 4 seulement, mais aussi une deuxième ouverture 14. Cette disposition améliore les conditions de mélange dans la chambre primaire de décantation 1 et augmente la section de passage entre les deux chambres primaire et secondaire de décantation 1 et 5.

Les deux plans déflecteurs 3a et 3b ne doivent pas nécessairement avoir la même inclinaison, mais le plan déflecteur 3b peut avoir une inclinaison de par exemple 0 à 10° moindre que celle du plan 3a dont la longueur est de préférence environ égale à celle des lamelles. Le plan déflecteur 3b est de telle longueur et disposition qu'il empêche les bulles de gaz formés dans la chambre primaire de décantation 1 de monter dans la chambre secondaire de décantation 5 et qu'il les force de prendre le chemin sous la hotte délimitée par le plan déflecteur 3A et une des parois 9 de la cuve.

La figure 3 montre une cuve de très grande section dans laquelle plusieurs hottes sont prévues délimitées, la première par une paroi verticale de la cuve 9 et le plan déflecteur incliné 3a, et les suivantes par des plans de fermeture 15 parallèles aux faisceaux de lames 7 et des plans déflecteurs inclinés 3a tous parallèles entr'eux. On parvient ainsi de réduire sensiblement l'espace mal utilisé de forme triangulaire entre la dernière lamelle du faisceau 7 à la droite sur la figure 3
et la paroi verticale 9 de la cuve. Dans l'exemple représenté, trois
modules composés chacun d'une hotte et d'un faisceau de lamelles sont
montrés. Le nombre de ces modules et leurs dimensions sont déterminés en
fonction de la vitesse de sédimentation du catalyseur chimique ou biologique dont on désire éviter qu'il soit entraîné vers les tuyauteries
d'évacuation. Par contre, si le liquide réactif contient des matières en
suspension non solubilisées, on a avantage de s'arranger pour que ces
matières en suspension ne s'accumulent pas de manière prohibitive dans
la cuve et soient évacuées avec le liquide effluent. En ce qui concerne
le recyclage du liquide effluent, il est possible aussi, en lieu et
place du liquide, de recycler du gaz ou un mélange de gaz et de liquide.

La cuve de réaction décrite convient particulièrement bien à un
processus microbiologique anaérobie en vue de la production de méthane,
mais n'est pas limité à cette application et peut être utilisé à d'autres processus microbiologiques ou même physico-chimiques.

REVENDICATIONS.

1.     Cuve de réaction  comprenant une tuyauterie d'amenée (13) d'un liquide réactif au bas d'une chambre primaire de décantation (1) surmontant   un lit (2) d'un catalysateur physico-chimique ou biologique, au moins un plan déflecteur inclié (3a,3b) faisant partie d'une hotte collectrice de gaz et recouvrant partiellement la chambre primaire de décantation (1), une chambre secondaire de décantation (5) adossée à au moins un des plans déflecteurs inclinés (3a, 3b) et une tuyauterie d'évacuation (6) dans la partie supérieure de la chambre secondaire de décantation (5),

caractérisée en ce que la chambre secondaire de décantation (5) est subdivisée en un grand nombre d'espaces inclinées de faible hauteur obtenus au moyen d'au moins un faisceau de lames inclinées (7) conduisant le liquide réactif vers le haut dans au moins un espace collecteur relié à la tuyauterie d'évacuation (6) et en ce qu'au moins un déflecteur (3b,12) incliné est disposé en dessous des ouvertures d'entrée de dits espaces inclinées.

2.   Cuve de réaction suivant la revendication 1, caractérisée en ce que le plan déflecteur incliné (3a, 3b) faisant partie d'une hotte collectrice de gaz est un seul et unique plan recouvrant la chambre primaire de décantation (1) sauf à l'endroit d'un déflecteur inférieur (12).

3.   Cuve de réaction suivant la revendication 1, caractérisée en ce que les plans déflecteurs inclinés (3a, 3b) faisant partie d'une hotte collectrice de gaz sont deux plans séparés l'un de l'autre par une ouverture (14) recouvrant ensemble, chacun une partie de la chambre primaire de décantation (1) sauf à l'endroit d'un déflecteur inférieur (12).

4.   Cuve de réaction suivant la revendication 1, caractérisée en ce que plusieurs plans déflecteurs inclinés (3a, 3b) forment soit avec une paroi (9) de la cuve soit avec un plan de séparation (15) des hottes collectrices de gaz et recouvrent, ensemble avec un déflecteur inférieur (12) les parties de la chambre primaire de décantation (1) disposées en dessous des ouvertures des espaces inclinées définies par les faisceaux de lames (7), mais ne recouvrent pas les ouvertures inférieures des dites hottes.

5.   Cuve de réaction suivant une des revendications précédentes , caractérisée en ce que les plans déflecteurs inclinés (3a; 3a, 3b) auxquels

39.19/1843.

sont adossées les faisceaux des lames (7) incluent avec l'horizontale un angle de 50 à 65°.

6.   Cuve de réaction suivvant une des revendications précédentes, caractérisée en ce que les plans déflecteurs inclinés (3b) non accomgnés d'un faisceau de lames incluent avec l'horizontale un angle de 40 à 65°.

7.   Cuve de réaction suivant une des revendications précédentes, caractérisée en ce que la longueur des lamelles du faisceau 7 et l'écartement entre celles-ci est choisie en fonction d'un diamètre de coupure de des particules et de la vitesse du liquide réactif dans les espaces entre les lamelles.

Fig.1

Fig.2

0061228

Fig.3